# EUROPEAN PATENT APPLICATION

(11) **EP 2 873 421 A1**
(43) Date of publication of application: **20.05.2015**
(21) Application number: 13782317.5
(22) Date of filing: 25.03.2013
(51) Int. Cl.: A61K 36/53, A61K 36/61, A61K 36/54, A61K 36/185, A61K 36/534, A61P 31/00, A61P 17/00

(54) **METHOD FOR PRODUCING A VITAL-ORGAN-REGENERATIVE PRODUCT AND BY-PRODUCT FOR CUTANEOUS USE**

(30) Priority: 24.04.2012 ES 201230607
(71) Applicant: Carballo de Sales, Manuel, 24100 Leon (ES)
(72) Inventor: Carballo de Sales, Manuel, 24100 Leon (ES)
(74) Representative: Lahidalga de Careaga, Jose Luis
(86) International application number: PCT/ES2013/070193
(87) International publication number: WO 2013/160505

(57) **Abstract**

A procedure and extraction of a regenerative product and a byproduct for cutaneous use consisting of an infusion based on one litre of water boiled at between 95º and 100º centigrade and where, for said quantity of water, the following products must be added, quantified by weight:

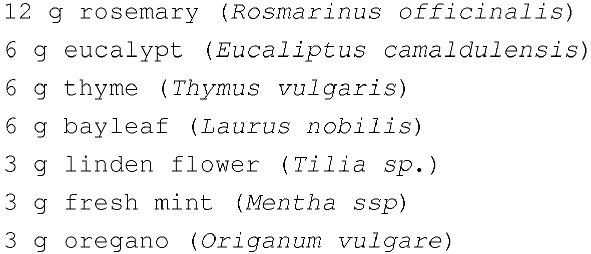

## Description

A patent of invention for a procedure and extraction of a regenerative product and a byproduct for cutaneous use consisting of an infusion of various natural products functioning as a natural antibiotic, immediately effective and with no side-effects, where the residual byproduct of said infusion offers a series of regenerative qualities for cutaneous use.

### FIELD OF THE INVENTION

The field of the invention is that of natural medicine and the manufacture of natural antibiotics and homeopathic products.

### BACKGROUND OF THE INVENTION

Antibiotics are chemical compounds used to eliminate or inhibit growth of infectious organisms.

Increasing bacterial resistance to chemical antibiotics and side-effects arising from their misuse means that they fail ever more often to obtain the desired effect.

While antibiotics used rationally are at times indispensable in medical practice, their abuse is producing generations of resistant bacteria; infections worsen and the patient is not cured.

Indeed, "indestructible" bacteria have been detected, which are unaffected by any known chemical antibiotic.

Medical science has warned of the massive and unthinking use of chemical antibiotics which have become not just chemical products devoid of effectiveness but also sometimes have clearly harmful effects.

All sorts of reactions may occur, from the slightest such as general discomfort, stomach irritation, diarrhea, etc., to those of a more serious nature such as deafness or irreversible kidney damage.

Natural antibiotics on the other hand are compounds from the plant world which are also able to eliminate or inhibit growth of infectious organisms.

For this reason they are able to prevent or cure many health conditions.

Unlike synthetic antibiotics produced in the laboratory, natural antibiotics obtained directly from different plant varieties offer at least the following benefits;
.- They have no side-effects.
.- They respect the microorganisms which are beneficial for the body, such as those for example needed to maintain the intestinal flora.
.- They offer no danger from accumulation.
.- They are economical, and easily acquired.

Some are known on the market, made from garlic *(Allium sativum*), onion *(Allium cepa*), echinacea *(Echinacea angustifolia),* ginger, *(Zingiber officinale*), mint *(Mentha ssp),* rosemary *(Rosmarinus officinalis),* linden flower *(Tilia sp),* thyme *(Thymus vulgaris)* or the various types of medicinal mushrooms.

However, the inventor is unaware of any preparation containing the elements described in these specifications nor in the proportions in the invention.

### DESCRIPTION OF THE INVENTION

Procedure and extraction of a regenerative product and a byproduct for cutaneous use consisting of an infusion of various natural products functioning as a natural antibiotic, immediately effective and with no side-effects, where the residual byproduct of said infusion offers a series of regenerative qualities for cutaneous use, particularly indicated for the regeneration of vital organs which have degenerated because of conditions such as leukemia and Alzheimer and other degenerative cellular processes.

The procedure in the invention consists of an infusion whose description in terms of volume can be said to be based on one litre of water boiled at between 95º and 100º centigrade.

With this quantity of water, the following products must be added, quantified by weight:

| | |
|---|---|
| 12 g | rosemary *(Rosmarinus officinalis*) |
| 6 g | eucalypt *(Eucaliptus camaldulensis*) |
| 6 g | thyme *(Thymus vulgaris)* |
| 6 g | bay *(Laurus nobilis)* |
| 3 g | linden flower *(Tilia sp.)* |
| 3 g | fresh mint *(Mentha ssp)* |
| 3 g | oregano *(Origanum vulgare)* |

This infusion is allowed to macerate for 3 hours, and yields a liquid which is filtered and considered to a natural antibiotic.

If said product is macerated for at least 16 hours, a liquid is obtained with the same characteristics as the above, and special cutaneous regenerative properties.

Having sufficiently described the nature of the invention and its practical implementation, it must be recorded that the specifications indicated above and represented in the attached drawings may be modified in detail provided that this does not alter their fundamental principles established in the previous paragraphs and summarised in the following claims.

## Claims

1. Procedure and extraction of a regenerative product and byproduct for cutaneous use consisting of an infusion of various natural products functioning as a natural antibiotic with regenerative qualities, and especially indicated for the regeneration of vital organs which have degenerated as a consequence of illness and other cellular degenerative processes, characterised essentially because the procedure in the invention consists of an infusion based on one litre of water boiled at between 95º and 100º centigrade and where, for said quantity of water, the following products must be added, quantified by weight:
| | |
|---|---|
| 12 g | rosemary *(Rosmarinus officinalis*) |
| 6 g | eucalypt *(Eucaliptus camaldulensis*) |
| 6 g | thyme *(Thymus vulgaris)* |
| 6 g | bayleaf *(Laurus nobilis)* |
| 3 g | linden flower *(Tilia sp.)* |
| 3 g | fresh mint *(Mentha ssp)* |
| 3 g | oregano *(Origanum vulgare)* |
This infusion is allowed to macerate for 3 hours, and yields a liquid which is filtered and considered to a natural antibiotic.

2. Procedure and extraction of a regenerative product and byproduct for cutaneous use consisting of an infusion of various natural products functioning as a natural antibiotic with regenerative qualities and especially indicated for the regeneration of vital organs which have degenerated as a consequence of illness and other cellular degenerative processes, as set forth in claim 1 and characterised because, if the mixture obtained according to the previous claim is allowed to macerate a minimum of 16 hours, a product with the same antibiotic characteristics is obtained, but for cutaneous use.
